(19) Europäisches Patentamt
European Patent Office
Office européen des brevets

(11) Veröffentlichungsnummer: **0 332 159**
**A1**

(12) # EUROPÄISCHE PATENTANMELDUNG

(21) Anmeldenummer: 89104064.4

(22) Anmeldetag: 08.03.89

(51) Int. Cl.⁴: C07D 207/00 , C07D 209/22 , C07D 223/16 , C07D 215/22 , C07D 235/06 , C07D 277/64 , C07D 279/16 , C07D 265/36 , C07D 241/42 , C07D 243/12

(30) Priorität: 10.03.88 DE 3807922

(43) Veröffentlichungstag der Anmeldung: 13.09.89 Patentblatt 89/37

(84) Benannte Vertragsstaaten: AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Leinert, Herbert, Dr. Ing.
Essigkamm 11
D-6148 Heppenheim(DE)
Erfinder: Tsaklakidis, Christos Dr. phil.
Weberstrasse 23
D-6940 Weinheim(DE)
Erfinder: Freund, Peter, Dr.
Hebelstrasse 83
D-6834 Ketsch(DE)

(54) **Substituierte Heterocyclen, Verfahren zu ihrer Herstellung sowie Arzneimittel, die diese Verbindungen enthalten.**

(57) Verbindungen der Formel I

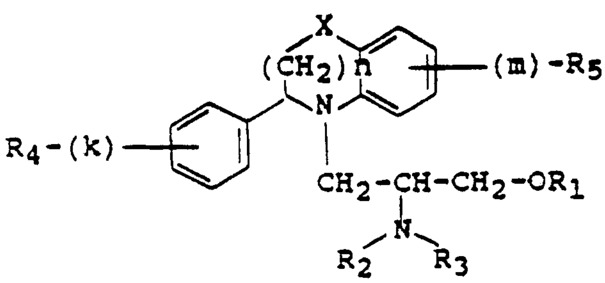

(I)

worin
$R_1$ einen $C_1$-$C_{12}$-Alkylrest, der durch einen Phenyl-, Naphthyl-oder einen $C_3$-$C_7$-Cycloalkylrest substituiert sein kann, einen $C_2$-$C_{12}$-Alkenylrest, der durch einen $C_3$-$C_7$-Cycloalkylrest oder einen Phenyl- oder Naphthyl substituiert sein kann, einen $C_3$-$C_7$-Cycloalkylrest oder einen gegebenenfalls substituierten mono- oder bicyclischen aromatischen Rest bedeutet,
wobei die Substituenten $C_1$-$C_4$-Alkyl, $C_1$-$C_4$-Alkoxy, Halogen, Carboxyl oder $C_1$-$C_4$-Alkoxycarbonyl sein können,
$R_2$ und $R_3$ einen gesättigten oder ungesättigten $C_1$-$C_6$-Alkylrest bedeuten oder zusammen mit dem Stickstoff einen gesättigten oder ungesättigten Ring bilden, der noch weitere Heteroatome erhalten kann und gegebenenfalls durch eine niedrige $C_1$-$C_6$ Alkylgruppe, eine oder mehrere $C_1$-$C_6$ Alkoxygruppen, durch eine oder mehrere Hydroxylgruppen oder ein Sauerstoffatom substituiert sein kann,

EP 0 332 159 A1

X eine Methylengruppe, ein Sauerstoffatom, ein Schwefelatom, die Sulfoxidgruppe, die Sulfonylgruppe oder die Gruppe $NR_6$ bedeutet, wobei $R_6$ Wasserstoff, $C_1$-$C_4$ Alkyl oder Aralkyl bedeuten kann,
n die Bedeutung 0, 1 oder 2 besitzt,
k die Bedeutung 0, 1, 2 oder 3 besitzt,
$R_4$ Halogen, $C_1$-$C_6$ Alkyl, $C_1$-$C_6$ Alkoxy, Aralkoxy, Hydroxy, oder wenn k gleich 2 ist an benachbarten Kohlenstoffatomen $C_1$-$C_2$ Alkylendioxy bedeutet
m die Bedeutung 0, 1, 2 oder 3 besitzt und
$R_5$ Halogen, Alkyl, Alkoxy, Aralkoxy, Hydroxy oder wenn m gleich 2 ist an benachbarten Kohlenstoffatomen Alkylendioxy bedeutet.
sowie deren pharmakolgisch verträglichen Salze, Verfahren zu ihrer Herstellung und Arzneimittel, die diese Verbindungen enthalten, zur Behandlung von Herz- und Kreislauferkrankungen.

# Substituierte Heterocyclen, Verfahren zu ihrer Herstellung sowie Arzneimittel, die diese Verbindungen enthalten

Die vorliegende Erfindung betrifft substituierte Heterocyclen, deren pharmakologisch verträgliche Salze, ihre Herstellung sowie diese enthaltende Arzneimittel.

Gegenstand der Erfindung sind substituierte Heterocyclen der allgemeinen Formel I

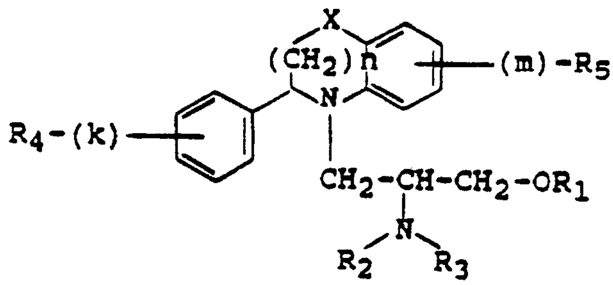

(I)

worin

$R_1$ einen geradkettigen oder verzweigten $C_1$-$C_{12}$-Alkylrest, der durch einen Phenyl-, Naphthyl- oder einen gegebenenfalls durch $C_1$-$C_4$-Alkyl substituierten $C_3$-$C_7$-Cycloalkylrest substituiert sein kann, einen geradkettigen oder verzweigten $C_2$-$C_{12}$-Alkylrest, der durch einen $C_3$-$C_7$-Cycloalkylrest oder einen Phenyl- oder Naphthyl substituiert sein kann, einen $C_3$-$C_7$-Cycloalkylrest oder einen unsubstituierten oder ein- oder mehrfach substituierten mono- oder bicyclischen aromatischen Rest bedeutet,

wobei die Substituenten $C_1$-$C_4$-Alkyl, $C_1$-$C_4$-Alkoxy, Halogen, Carboxyl oder $C_1$-$C_4$-Alkoxycarbonyl sein können,

$R_2$ und $R_3$ gleich oder verschieden sein können und einen geradkettigen, verzweigten, gesättigten oder ungesättigten $C_1$-$C_6$ Alkylrest bedeuten oder zusammen mit dem Stickstoff einen gesättigten oder ungesättigten Ring bilden, der noch weitere Heteroatome enthalten kann und gegebenenfalls durch eine niedrige $C_1$-$C_6$ Alkylgruppe, eine oder mehrere $C_1$-$C_6$ Alkoxygruppen, durch eine oder mehrere Hydroxylgruppen oder ein Sauerstoffatom substituiert sein kann,

X eine Methylengruppe, ein Sauerstoffatom, ein Schwefelatom, die Sulfoxidgruppe, die Sulfonylgruppe oder die Gruppe $NR_6$ bedeutet, wobei $R_6$ Wasserstoff, $C_1$-$C_4$ Alkyl oder Aralkyl bedeuten kann,

n die Bedeutung 0, 1 oder 2 besitzt,

k die Bedeutung 0, 1, 2 oder 3 besitzt,

$R_4$ Halogen, $C_1$-$C_6$ Alkyl, $C_1$-$C_6$ Alkoxy, Aralkoxy, Hydroxy oder wenn k gleich 2 ist an benachbarten Kohlenstoffatomen $C_1$-$C_2$ Alkylendioxy bedeutet

m die Bedeutung 0, 1, 2 oder 3 besitzt und

$R_5$ Halogen, $C_1$-$C_4$ Alkyl, $C_1$-$C_6$ Alkoxy, Aralkoxy, Hydroxy oder wenn m = 2 ist an benachbarten Kohlenstoffatomen $C_1$-$C_2$ Alkylendioxy bedeutet.

sowie deren pharmakolgisch verträglichen Salze und optische Isomere.

Der $C_1$-$C_{12}$ Alkylrest $R_1$ bedeutet vorzugsweise Methyl, Ethyl, Propyl, Isopropyl, n-Butyl, Isobutyl, Isoamyl, Isohexyl, n-Hexyl, n-Octyl, n-Dodecyl, insbesondere Isobutyl, Isoamyl, Isohexyl und Neopentyl.

Der $C_3$-$C_7$ Cycloalkylrest $R_1$ bedeutet in der Regel Cyclopropyl, Cyclopentyl und Cyclohexyl, wobei der Cycloalkylrest noch einen zusätzlichen Alkylrest wie Methyl, Ethyl oder Propyl besitzen kann.

Wenn der Alkylrest substituiert ist, kommen insbesondere der Cyclopentyl-, der Cyclohexyl, der Phenyl- bzw. Naphthyl-Rest in Frage, wobei der Cyclopentyl bzw. der Cyclohexyl-Rest noch zusätzlich eine Alkylrest wie Methyl, Ethyl oder Propyl besitzen kann. Bevorzugt ist der 1-Methylcyclohexylmethyl-Rest.

Die $C_2$-$C_{12}$ Alkenylreste $R_1$ sind vorzugsweise Allyl, Methylallyl, Isopentenyle, Hexenyle, Octenyle und Dodecenyle. Ein substituierter Rest stellt z.B. der Cinnamylrest dar. Stellt der Rest $R_1$ eine mono- oder bicyclischen aromatischen Rest dar, versteht man darunter Phenyl, Naphthyl, Indanyl, Indenyl und Tetralinyl. Hierin bedeuten $C_1$-$C_4$-Alkyl, Methyl, Ethyl, Propyl, Isopropyl, Butyl oder t-Butyl, $C_1$-$C_4$-Alkoxy, Methoxy, Ethoxy, Propoxy, Isopropoxy, Butoxy oder t-Butoxy. Halogen bedeutet Chlor, Brom oder Fluor. Unter $C_1$-$C_4$ Alkoxycarbonyl versteht man einen Methylester, Ethylester, Propylester, Butylester oder t-Butylester.

$R_2$ und $R_3$ bedeuten vorzugsweise Methyl, Ethyl, Propyl, Allyl oder Methallyl. Ringe, die $R_2$ und $R_3$ zusammen mit dem Stickstoffatom, an das sie gebunden sind, bilden können, sind vorzugsweise der

Pyrrolidin-, der Pyrrolin-und der Piperidinring, insbesondere der Pyrrolidinring. Die Heteroatome, die die Ringe enthalten können, sind Stickstoff, Schwefel oder Sauerstoff. Es sind hierunter Ringe wie z.B. Piperazin, Morpholin und Thiomorpholin zu verstehen. Substituenten der vorstehend genannten Ringe sind insbesondere Hydroxylgruppen, $C_1$-$C_3$ Alkyl- und $C_1$-$C_3$ Alkoxygruppen, wie z.B. Methyl, Ethyl, Propyl oder Methoxy, Ethoxy oder Propoxy. Der Sauerstoff-Substituent stellt in der Regel mit dem C-Atom, an das er gebunden ist, eine Carbonylgruppe dar. Entsprechende Ringe sind z.B. der Pyrrolidinon- und der Piperidinon-Ring.

Wenn $R_4$ Halogen bedeutet, versteht man darunter Chlor, Brom oder Fluor. Alkyl bedeutet $C_1$-$C_6$ Alkyl, vorzugsweise $C_1$-$C_4$ Alkyl wie Methyl, Ethyl, Propyl, Butyl. Alkoxy bedeutet $C_1$-$C_6$-Alkoxy, vorzugsweise $C_1$-$C_4$-Alkoxy wie Methoxy, Ethoxy, n-Propoxy, Isopropoxy, n-Butoxy, Isobutoxy oder t-Butoxy. Unter Aralkoxy versteht man vorzugsweise die Benzyloxygruppe. Wenn k die Zahl 2 an benachbarten Kohlenstoffatomen bedeutet, versteht man unter $R_4$ die Methylendioxy- oder Ethylendioxygruppe.

k ist 0 bis 3, was bedeutet, daß entweder kein Substituent vorhanden ist oder die Zahl der Substituenten zwischen 1 und 3 variieren kann, wobei alle möglichen Kombination der Substituenten unabhängig voneinander möglich sind. Bevorzugt sind die Zahlen 0 oder 1.

Wenn $R_5$ Halogen bedeutet, versteht man darunter Chlor, Brom oder Fluor. $C_1$-$C_4$ Alkyl bedeutet in der Regel Methyl, Ethyl, Propyl, Butyl. $C_1$-$C_6$ Alkoxy bedeutet Methoxy, Ethoxy, n-Propoxy, Isopropoxy, n-Butoxy, Isobutoxy oder t-Butoxy. Unter Aralkoxy versteht man vorzugsweise die Benzyloxygruppe. Wenn m die Zahl 2 an benachbarten Kohlenstoffatomen bedeutet, versteht man unter $R_5$ die Methylendioxy- oder Ethylendioxygruppe.

m ist 0 bis 3, was bedeutet, daß entweder kein Substituent vorhanden ist, oder die Zahl der Substituenten zwischen 1 und 3 variieren kann, wobei alle möglichen Kombinationen der Substituenten unabhängig voneinander möglich sind. Bevorzugt sind die Zahlen 0 oder 1.

Wenn $R_6$ $C_1$-$C_4$ Alkyl bedeutet, versteht man darunter Methyl, Ethyl, Propyl oder Butyl. Aralkyl bedeutet Benzyl oder Phenethyl.

Bevorzugt sind Verbindungen der Formel I, in der

R· Isobutyl, Isoamyl, Isohexyl, Neopentyl, 1-Methylcyclohexylmethyl, Allyl, Methallyl, Isopentyl, Hexenyl, Octenyl, Dodecenyl oder Cinnamyl,

$R_2$ und $R_3$ jeweils Ethyl oder zusammen mit dem Stickstoffatom, an das sie gebunden sind, einen Pyrrolidin-Ring bilden,

X eine Methylengruppe, ein Sauerstoff- oder Schwefelatom, oder die Gruppe NH,

K die Zahlen 0 oder 1,

m die Zahlen 0 oder 1,

n die Zahlen 0 oder 1,

$R_4$ Methoxy und

$R_5$ Methoxy

bedeuten.

Die erfindungsgemäßen Verbindungen der allgemeinen Formel I können in an sich bekannter Weise dargestellt werden, daß man

(a) eine Verbindung der allgemeinen Formel II,

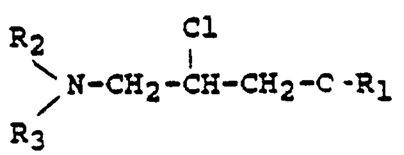

(II)

in der $R_1$, $R_2$, und $R_3$ die oben genannten Bedeutungen besitzen, mit einer Verbindung der allgemeinen Formel III,

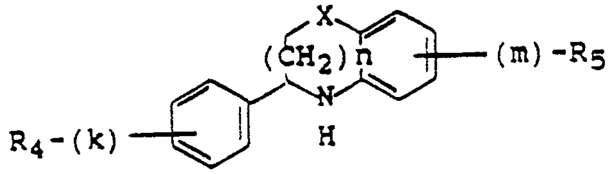

(III)

in der $R_4$, $R_5$, k, m und X die oben genannten Bedeutungen besitzen, umsetzt mit der Ausnahme, daß man, wenn die beiden Reste $R_2$ und $R_3$ zusammen einen durch eine oder zwei Hydroxylgruppen substituierten Ring bilden oder einer der Reste $R_4$ oder $R_5$ eine Hydroxylgruppe darstellt, diese vor der Umsetzung schütt und nach beendeter Reaktion wieder abspaltet. Als Schutzgruppen kommen z.B. in Frage Benzyloxygruppen oder Methoxy-methylenoxy-gruppen.

oder

b) eine Verbindung der allgemeinen Formel IV,

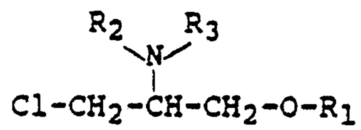

(IV)

in der $R_1$, $R_2$, und $R_3$ die oben genannten Bedeutungen besitzen, mit einer Verbindung der allgemeinen Formel III umsetzt,
wobei die gleichen Ausnahmen, wie unter a) beschrieben, gelten.

oder

c) eine Verbindung der allgemeinen Formel V,

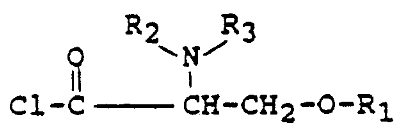

(V)

in der $R_1$, $R_2$, und $R_3$ die oben genannten Bedeutungen besitzen mit einer Verbindung der allgemeinen Formel III umsetzt und die erhaltene Verbindung der allgemeinen Formel VI,

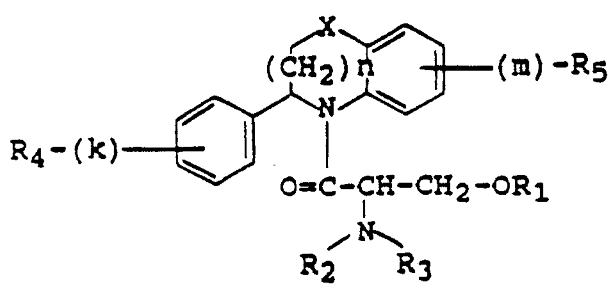

(VI)

in der $R_1$, $R_2$, $R_3$, $R_4$, $R_5$, X, k, m und n die oben genannten Bedeutungen, einer Reduktion mit einem komplexen Hydrid unterwirft.

Die Umsetzung der Verbindungen der allgemeinen Formel II mit einer Verbindung der allgemeinen Formel III zu den erfindungsgemäßen Verbindungen der allgemeinen Formel I erfolgt, in an sich bekannter Weise, in einem inerten Lösungsmittel, wie Toluol, Xylol oder Dimethylformamid bei Temperaturen zwischen 40 °C und Rückflußtemperatur des Lösungsmittels in Gegenwart eines alkalischen Kondensationsmittels, z.B. Natriumhydrid oder Natriumamid.

Die Verbindungen der allgemeinen Formel II können z.B. dargestellt werden, indem man eine Verbindung der allgemeinen Formel VII,

$R_1OH$ (VII)

in der $R_1$ die angegebene Bedeutung besitzt, mit Epichlorhydrin in Gegenwart von Natronlauge und eines Phasentransferkatalysators z.B. Tetrabutylamminiumbromid zur Reaktion bringt bei Temperaturen zwischen Rautemperatur und 50 °C und die erhaltene Verbindungen der allgemeinen Formel VIII,

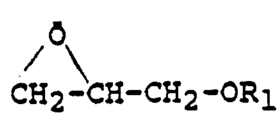

(VIII)

mit einem Amin der allgemeinen Foreml IX,

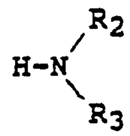

(IX)

in der $R_2$ und $R_3$ die angegebenen Bedeutungen besitzen, in an sich bekannter Weise, in einem Lösungsmittel z.B. Ethanol oder Propanol bei Temperaturen zwischen Raumtemperatur und Rückflußtemperatur des Lösungsmittels zur Reaktion bringt und die erhaltene Verbindung der allgermeinen Formel X,

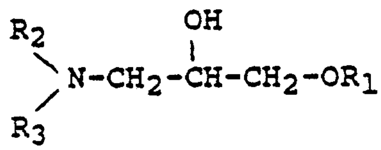

(X)

in einem inerten Lösungsmittel z.B. Dichloroethan mit einem Chlorierungsittel z.B. Thionylchlorid zu einer Verbindung der allgemeinen Formel II umsetzt.

Die Umsetzung der Verbindungen der allgemeinen Formel IV mit Verbindungen der allgemeinen Formel III zu den erfindungsgemäßen Verbindungen der allgemeinen Formel I erfolgt in einem inerten Lösungsmittel wie Toluol oder Xylol oder Dimethylformamid bei Temperaturen zwischen 40 °C und Rückflußtemperatur des Lösungsmittels in Gegenwart eines alkalischen Kondensationsmittels wie Natriumhydrid oder Natriumamid.

Die Verbindungen der allgemeinen Formel IV können dargestellt werden, daß man eine Verbindung der allgemeinen Formel XI,

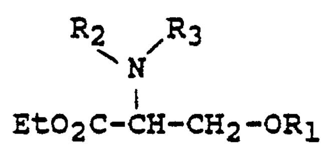

(XI)

in der $R_1$, $R_2$ und $R_3$ die angegebenen Bedeutungen besitzen mit einem komplexen Hydrid, z.B. Lithiumaluminiumhydrid, in einem inerten Lösungsmittel z.B. Ether oder Tetrahydrofuran in an sich bekannter Weise zu einer Verbindung der allgemeinen Formel XII,

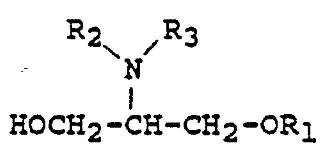

(XII)

reduziert und diese in einem inerten Lösungsmittel, z.B. Dichlorethan mit einem Chlorierungsmittel z.B. Thionylchlorid zu einer Verbindung der allgemeinen Formel IV umsetzt.

Die Ausgangsverbindungen der allgemeinen Formel XI können nach einem in der deutschen Patentschrift DE-2802864 beschriebenen Verfahren hergestellt werden.

Die Umsetzung der Verbindungen der allgemeinen Formel V mit einer Verbindung der allgemeinen Formel III zu einer Verbindung der allgemeinen Formel VI erfolgt nach Lit. bekannten Verfahren in einem inerten Lösungsmittel, z.B. Methylenchlorid in Gegenwart eines säurebindenden Mittels z.B. Triethylamin oder z.B. in Pyridin. Die Reduktion der Verbindungen der allgemeinen Formel VI zu den erfindungsgemäßen Verbindungen der allgemeinen Formel I erfolgt in einem inerten Lösungsmittel z.B. Ether oder Tetrahydrofuran mit einem komplexen Hydrid z.B. Lithiumaluminiumhydrid.

Die Verbindungen der allgemeinen Formel V können hergestellt werden, indem man eine Verbindung der allgemeinen Formel XI einer Hydrolyse unterwirft und die erhaltene Verbindung der allgemeinen Formel

XIII,

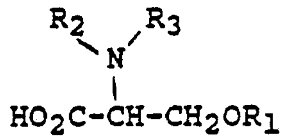

(XIII)

in der $R_1$, $R_2$ und $R_3$ die angegebenen Bedeutungen besitzen mit einem Halogenierungsmittel in einem inerten Lösungsmittel, z.B. Methylenchlorid, Dichlorethan oder Benzol zur Reaktion bringt.

Die als Ausgangsmaterialien verwendeten Alkohole der allgemeinen Formel I,

$R_1OH$ (I)

sind käuflich oder in der Literatur beschrieben.

Die Amine der allgemeinen Formel IX,

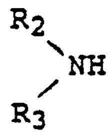

(IX)

sind käuflich oder in der Literatur beschrieben.

Die Verbindungen der allgemeinen Formel III sind in der Literatur beschrieben.

Die erfindungsgemäßen Verbindungen der allgemeinen Formel I besitzen asymmetrische Kohlenstoffatome.

Gegenstand der Erfindung sind daher auch Diastereomerengemische und sämtliche optisch aktive Formen der erfindungsgemäßen Verbindungen der allgemeinen Formel I.

Die Trennung der Diastereomerengemische erfolgt z.B. durch chromatographische Methoden an einer Kieselgelsäule. Die anschließende Trennung der Enantiomerengemische erfolgt nach bekannten Methoden über diastereomeren Salze.

Optisch aktive Säuren, die zur Racematenspaltung, eingesetzt werden können, sind z.B. Weinsäure, Äpfelsäure, Camphersäure, Camphersulfonsäure oder Dibenzoylweinsäure.

Zur Überführung der Verbindungen der allgemeine Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Salicylsäure, Citronensäure, Benzoesäure, Naphthoesäure, o-Acetoxybenzoesäure, Adipinsäure, Maleinsäure oder Oxalsäure um.

Die erfindungsgemäßen Verbindungen der allgemeinen Formel I besitzen wertvolle pharmakologische Eigenschaften. Sie zeichnen sich besonders durch eine gefäßrelaxierende Wirkung aus und können daher zur Therapie von Herz-/Kreislauf-Erkrankungen eingesetzt werden.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zu Anwendung, welches die bei Injektionslösungen üblichen Zusätze wie Stabilisierungsmittel, Lösungsvermittler oder Puffer enthält. Derartige Zusätze sind z.B. Tartrat- und Citrat-puffer, Ethanol, Komplexbildner (wie Ethylendiamintetraessigsäure und deren nicht-toxischen Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum, hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hoch molekulare Polymere (wie z.B. Polyethylenglykole), für orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Eine angemessene tägliche Dosis der aktiven Verbindung liegt zwischen 0,01 bis 50 mg/kg Köpergewicht, wobei diese in geeigneten Dosiseinheiten ein- oder mehrfach pro Tag verabreicht werden können.

Neben den nachfolgenden aufgeführten Beispielen sind insbesondere die folgenden Verbindungen im Sinne der Anmeldung bevorzugt:

1-[3-(2-Methyl-propyloxy)-2-(3-hydroxy-pyrrolidino)-propyl]-2-phenyl-indolin
1-[3-(2-Methyl-propyloxy)-2-(3,4-dihydroxy-pyrrolidino)-propyl]-2-phenyl-indolin
1-[3-(2-Methyl-propyloxy)-2-(3-methoxy-pyrrolidino)-propyl]-2-phenyl-indolin
1-[3-(2-Methyl-propyloxy)-2-(3,4-dimethoxy-pyrrolidino)-propyl]-2-phenyl-indolin
3,4-Dihydro-4-[3-(2-methyl-2-propenyl-1-oxy)-2-(3-hydroxy-pyrrolidino)-propyl]-3-phenyl-2H-1,4-benzoxazin
3,4-Dihydro-4-[3-(2-methyl-2-propenyl-1-oxy)-2-(3-methoxy-pyrrolidino)-propyl]-3-phenyl-2H-1,4-benzoxazin
3,4-Dihydro-4-[3-(2-methyl-2-propenyl-1-oxy)-2-(3,4-dimethoxy-pyrrolidino)-propyl]-3-phenyl-2H-1,4-benzoxazin
3,4-Dihydro-4-[3-(3-methyl-2-butenyl-1-oxy)-2-(3-methoxy-pyrrolidino)-propyl]-3-phenyl-2H-1,4-benzoxazin
3,4-Dihydro-4-[3-(3-methyl-2-butenyl-1-oxy)-2-(3,4-dimethoxy-pyrrolidino)-propyl]-3-phenyl-2H-1,4-benzoxazin
1-[3-(3-Methyl-3-butenyl-1-oxy)-2-(3-hydroxy-pyrrolidino)-propyl]-2-phenyl-1,2,3,4-tetrahydro-chinolin.
1-[3-(3-Methyl-3-butenyl-1-oxy)-2-(3,4-dihydroxy-pyrrolidino)-propyl]-2-phenyl-1,2,3,4-tetrahydro-chinolin.
1-[3-(3-Methyl-3-butenyl-1-oxy)-2-(3-methoxy-pyrrolidino)-propyl]-2-phenyl-1,2,3,4-tetrahydro-chinolin.
1-[3-(3-Methyl-3-butenyl-1-oxy)-2-(3,4-dimethoxy-pyrrolidino)-propyl]-2-phenyl-1,2,3,4-tetrahydro-chinolin.
3,4-Dihydro-4-[3-(2-methyl-3-butenyl-2-oxy)-2-(3-hydroxy-pyrrolidino)-propyl]-3-phenyl-2H-1,4-benzoxazin
3,4-Dihydro-4-[3-(2-methyl-3-butenyl-2-oxy)-2-(3,4-dihydroxy-pyrrolidino)-propyl]-3-phenyl-2H-1,4-benzoxazin
3,4-Dihydro-4-[3-(2-methyl-3-butenyl-2-oxy)-2-(3-methoxy-pyrrolidino)-propyl]-3-phenyl-2H-1,4-benzoxazin
3,4-Dihydro-4-[3-(2-methyl-3-butenyl-2-oxy)-2-(3,4-dimethoxy-pyrrolidino)-propyl]-3-phenyl-2H-1,4-benzoxazin
1-[3-((1-Methyl-cyclohexyl)-methoxy)-2-(3-methoxy-pyrrolidino)-propyl]-4-methyl-2-phenyl-1,2,3,4-tetrahydro-1,4-benzodiazin.
1-[3-((1-Methyl-cyclohexyl)-methoxy)-2-(3,4-dimethoxy-pyrrolidino)-propyl]-4-methyl-2-phenyl-1,2,3,4-tetrahydro-1,4-benzodiazin.
3,4-Dihydro-4-[3-(2,2-dimethyl-propyloxy)-2-(3-hydroxy-pyrrolidino)-propyl]-3-phenyl-2H-1,4-benzothiazin
3,4-Dihydro-4-[3-(2,2-dimethyl-propyloxy)-2-(3-methoxy-pyrrolidino)-propyl]-3-phenyl-2H-1,4-benzothiazin
3,4-Dihydro-4-[3-(2,2-dimethyl-propyloxy)-2-(3,4-dimethoxy-pyrrolidino)-propyl]-3-phenyl-2H-1,4-benzothazin
3,4-Dihydro-4-[3-(3-methyl-butyl-1-oxy)-2-pyrrolidino-propyl]-3-phenyl-2H-1,4-benzoxazin
3,4-Dihydro-4-[3-(4-methyl-pentyl-1-oxy)-2-pyrrolidino-propyl]-3-phenyl-2H-1,4-benzoxazin
3,4-Dihydro-4-[3-(3-methyl-butyl-1-oxy)-2-(3-methoxy-pyrrolidino)-propyl]-3-phenyl-2H-1,4-benzoxazin
3,4-Dihydro-4-[3-(3-methyl-butyl-1-oxy)-2-(3,4-dimethoxy-pyrrolidino)-propyl]-3-phenyl-2H-1,4-benzoxazin
3,4-Dihydro-4-[3-(3-methyl-butyl-1-oxy)-2-diethylamino-propyl]-3-phenyl-2H-1,4-benzoxazin
3,4-Dihydro-4-[3-(4-methyl-pentyl-1-oxy)-2-diethylamino-propyl]-3-phenyl-2H-1,4-benzoxazin
3,4-Dihydro-4-[3-(3-methyl-butyl-1-oxy)-2-pyrrolidino-propyl]-3-phenyl-2H-1,4-benzothiazin
1-[3-(4-Methyl-pentyl-1-oxy)-2-pyrrolidino-propyl]-2-phenyl-1,2,3,4-tetrahydro-chinolin.
1-[3-(3-Methyl-butyl-1-oxy)-2-diethylamino-propyl]-2-phenyl-1,2,3,4-tetrahydro-chinolin.
3,4-Dihydro-4-[3-(3-methyl-butyl-1-oxy)-2-diethylamino-propyl]-3-phenyl-2H-1,4-benzothiazin
1-(3-Phenoxy-2-pyrrolidino-propyl)-2-phenyl-indolin
3,4-Dihydro-4-(3-phenoxy-2-pyrrolidino-propyl]-3-phenyl-2H-1,4-benzoxazin
3,4-Dihydro-4-[3-(4-methoxy-phenoxy)-2-diethylamino-propyl]-3-phenyl-2H-1,4-benzoxazin
3,4-Dihydro-4-[3-(4-methoxy-phenoxy-2-pyrrolidino-propyl]-3-(4-methoxy-phenyl)-2H-1,4-benzoxazin
3,4-Dihydro-4-[3-(4-methoxy-phenoxy)-2-(3-methoxy-pyrrolidino)-propyl]-3-(4-methoxy-phenyl)-2H-1,4-benzoxazin
3,4-Dihydro-4-[3-(4-methoxy-phenoxy)-2-(3,4-dimethoxy-pyrrolidino)-propyl]-3-(4-methoxy-phenyl)-7-methoxy-2H-1,4-benzoxazin
1-(3-Benzyloxy-2-pyrrolidino-propyl)-2-phenyl-indolin
3,4-Dihydro-4-(3-benzyloxy-2-pyrrolidino-propyl)-3-phenyl-2H-1,4-benzoxazin
3,4-Dihydro-4-(3-benzyloxy-2-pyrrolidino-propyl)-3-phenyl-2H-1,4-benzothiazin
3,4-Dihydro-4-[3-(4-methoxy-benzyloxy)-2-diethylamino-propyl]-3-phenyl-2H-1,4-benzoxazin
3,4-Dihydro-4-[3-(4-methoxy-benzyloxy)-2-diethylamino-propyl]-3-(4-methoxy-phenyl)-2H-1,4-benzoxazin
3,4-Dihydro-4-(3-phenoxy-2-pyrrolidino-propyl)-3-(3-chlor-phenyl)-2H-1,4-benzothiazin
1-(3-Phenoxy-2-pyrrolidino-propyl)-2-(4-methyl-phenyl)-1,2,3,4-tetrahydrochinolin.
1-(3-Phenoxy-2-pyrrolidino-propyl)-2-(3,4-dimethoxy-phenyl)-indolin
3,4-Dihydro-4-(3-benzyloxy-2-pyrrolidino-propyl)-3-(4-methoxy-phenyl)-2H-1,4-benzoxazin
3,4-Dihydro-4-(3-benzyloxy-2-diethylamino-propyl)-3-(4-methoxy-phenyl)-2H-1,4-benzoxazin
3,4-Dihydro-4-(3-benzyloxy-2-pyrrolidino-propyl)-3-(3-chlor-phenyl)-2H-1,4-benzothiazin
1-(3-Benzyloxy-2-pyrrolidino-propyl)-2-(3,4-dimethoxy-phenyl)-indolin

3,4-Dihydro-4-[3-(2-phenyl-ethyloxy)-2-diethylamino-propyl]-3-phenyl-2H-1,4-benzoxazin
3,4-Dihydro-4-[3-(2-phenyl-ethyloxy)-2-pyrrolidino-propyl]-3-phenyl-2H-1,4-benzoxazin
3,4-Dihydro-4-[3-(3-phenyl-2-propenyl-1-oxy)-2-diethyl-amino-propyl]-3-phenyl-2H-1,4-benzoxazin
3,4-Dihydro-4-[3-(3-phenyl-2-propenyl-1-oxy)-2-pyrrolidino-propyl]-3-phenyl-2H-1,4-benzoxazin
3,4-Dihydro-4-[3-(3-phenyl-2-propenyl-1-oxy)-2-diethylamino-propyl]-3-(4-methoxy-phenyl)-2H-1,4-benzoxazin
3,4-Dihydro-4-[3-(3-phenyl-2-propenyl-1-oxy)-2-pyrrolidino-propyl]-3-(4-methoxy-phenyl)-2H-1,4-benzoxazin
3,4-Dihydro-4-[3-(2-methyl-propyloxy)-2-pyrrolidino-propyl]-3-(3,4-methylendioxy-phenyl)-2H-1,4-benzoxazin
3,4-Dihydro-4-[3-(2-methyl-propyloxy)-2-pyrrolidino-propyl]-3-(4-methoxy-phenyl)-2H-1,4-benzothiazin
1-[3-2-Methyl-propyloxy)-2-pyrrolidino-propyl]-2-(4-methoxy-phenyl)-4-methoxy-indolin
3,4-Dihydro-4-[3-(2-methyl-propyloxy)-2-pyrrolidino-propyl]-3-(3,4-dimethoxy-phenyl)-2H-1,4-benzoxazin
3,4-Dihydro-4-[3-(2-methyl-propyloxy)-2-diethylamino-propyl]-3-(3,4-dimethoxy-phenyl)-2H-1,4-benzoxazin
3,4-Dihydro-4-[3-(2-methyl-2-propenyl-1-oxy)-2-pyrrolidino-propyl]-3-(3,4-dimethoxy-phenyl)-2H-1,4-benzoxazin
3,4-Dihydro-4-[3-(2-methyl-2-propenyl-1-oxy)-2-diethylamino-propyl]-3-(3,4-dimethoxy-phenyl)-2H-1,4-benzoxazin.
3,4-Dihydro-4-[3-(2-methyl-2-propenyl-1-oxy)-2-(3-methoxy-pyrrolidino)-propyl]-3-(3,4-dimethoxy-phenyl)-2H-1,4-benzoxazin
3,4-Dihydro-4-[3-(2-methyl-propyloxy)-2-pyrrolidino-propyl]-3-(4-fluor-phenyl)-2H-1,4-benzoxazin
3,4-Dihydro-4-[3-(2-methyl-2-propenyl-1-oxy)-2-pyrrolidino-propyl]-3-(4-fluor-phenyl)-2H-1,4-benzoxazin
3,4-Dihydro-4-[3-(2,2-dimethyl-propyloxy)-2-diethylamino-propyl]-3-(4-fluor-phenyl)-2H-1,4-benzoxazin
3,4-Dihydro-4-[3-((1-methylcyclohexyl)-methoxy)-2-(3-hydroxy-pyrrolidino)-propyl]-3-(4-fluor-phenyl)-2H-1,4-benzoxazin
3,4-Dihydro-4-[3-(2-methyl-propyloxy)-2-pyrrolidino-propyl]-3-(3,4,5-trimethoxy-phenyl)-2H-1,4-benzoxazin
3,4-Dihydro-4-[3-(2-methyl-2-propenyl-1-oxy)-2-diethylamino-propyl]-3-(3,4,5-trimethoxy-phenyl)-2H-1,4-benzoxazin
3,4-Dihydro-4-[3-(2-methyl-3-butenyl-2-oxy)-2-pyrrolidino-propyl]-3-(3,4,5-trimethoxy-phenyl)-2H-1,4-benzoxazin
3,4-Dihydro-4-[3-(2-methyl-propoxy)-2-pyrrolidino-propyl]-3-(3-methoxy-phenyl)-2H-1,4-benzoxazin
3,4-Dihydro-4-[3-(2-methyl-2-propenyl-1-oxy)-2-pyrrolidino-propyl]-3-(2-methoxy-phenyl)-2H-1,4-benzoxazin
3,4-Dihydro-4-[3-(3-methyl-2-butenyl-1-oxy)-2-diethylamino-propyl]-3-(2-methoxy-phenyl)-2H-1,4-benzoxazin
3,4-Dihydro-4-[3-(2,2-dimethyl-propyloxy)-2-(3-methoxy-pyrrolidino)-propyl]-3-(2-methoxy-phenyl)-2H-1,4-benzoxazin
3,4-Dihydro-4-[3-(2-methyl-propoxy)-2-pyrrolidino-propyl]-3-(3-methoxy-phenyl)-2H-1,4-benzoxazin
3,4-Dihydro-4-[3-(2-methyl-2-propenyl-1-oxy)-2-pyrrolidino-propyl]-3-(3-methoxy-phenyl)-2H-1,4-benzoxazin
3,4-Dihydro-4-[3-(3-methyl-2-butenyl-1-oxy)-2-diethylamino-propyl]-3-(3-methoxy-phenyl)-2H-1,4-benzoxazin
3,4-Dihydro-4-[3-(2,2-dimethyl-propyloxy)-2-pyrrolidino-propyl]-3-(3-methoxy-phenyl)-2H-1,4-benzoxazin
3,4-Dihydro-4-[3-(2-methyl-propoxy)-2-(3-methoxy-pyrrolidino-propyl]-3-(3-methoxy-phenyl)-2H-1,4-benzoxazin
3,4-Dihydro-4-[3-(2-methyl-2-propenyl-1-oxy)-2-(3-methoxy-pyrrolidino-propyl]-3-(3-methoxy-phenyl)-2H-1,4-benzoxazin
3,4-Dihydro-4-(3-phenoxy-2-pyrrolidino-propyl)-3-(3-methoxy-phenyl)-2H-1,4-benzoxazin
3,4-Dihydro-4-(3-benzyloxy-2-pyrrolidino-propyl)-3-(3-methoxy-phenyl)-2H-1,4-benzoxazin
3,4-Dihydro-4-[3-(2-phenyl-ethyloxy)-2-(3-methoxy-pyrrolidino)-propyl]-3-(3-methoxy-phenyl)-2H-1,4-benzoxazin
3,4-Dihydro-4-[3-(2-methyl-2-propenyl-1-oxy)-2-pyrrolidino-propyl]-3-(4-methoxy-phenyl)-2H-1,4-benzoxazin
3,4-Dihydro-4-[3-(2-methyl-2-propenyl-1-oxy)-2-diethylamino-propyl]-3-(4-methoxy-phenyl)-2H-1,4-benzoxazin
3,4-Dihydro-4-[3-(2,2-dimethyl-propyloxy)-2-diethylamino-propyl]-3-(4-methoxy-phenyl)-2H-1,4-benzoxazin
3,4-Dihydro-4-[3-(2-methyl-2-propenyl-1-oxy)-2-morpholino-propyl]-3-(4-methoxy-phenyl)-2H-1,4-benzoxazin
3,4-Dihydro-4-[3-(2-methyl-3-butenyl-1-oxy)-2-diethylamino-propyl]-3-(4-methoxy-phenyl)-2H-1,4-benzoxazin
3,4-Dihydro-4-[3-(3-methyl-2-butenyl-1-oxy)-2-morpholino-propyl]-3-(4-methoxy-phenyl)-2H-1,4-benzoxazin
3,4-Dihydro-4-[3-(2-methyl-propyloxy)-2-piperidino-propyl]-3-(4-methoxy-phenyl)-2H-1,4-benzoxazin
3,4-Dihydro-4-[3-(2-methyl-propoxy)-2-pyrrolidino-propyl]-3-(4-hydroxy-phenyl)-2H-1,4-benzoxazin
3,4-Dihydro-4-[3-(2-methyl-propoxy)-2-(3-methoxy-pyrrolidino)-propyl]-3-(4-hydroxy-phenyl)-2H-1,4-benzoxazin
3,4-Dihydro-4-(3-phenoxy-2-diethylamino-propyl)-3-4-(hydroxy-phenyl)-2H-1,4-benzoxazin
3,4-Dihydro-4-(3-benzyloxy-2-morpholino-propyl)-3-(4-hydroxy-phenyl)-2H-1,4-benzoxazin

3,4-Dihydro-4-[3-(2-phenyl-ethoxy)-2-piperidino-propyl)-3-(4-hydroxy-phenyl)-2H-1,4-benzoxazin
3,4-Dihydro-4-[3-(2-methyl-propoxy)-2-pyrrolidino-propyl]-3-(4-allyloxy-phenyl)-2H-1,4-benzoxazin
3,4-Dihydro-4-(3-phenoxy-2-diethylamino-propyl)-3-(4-allyloxy-phenyl)-2H-1,4-benzoxazin
3,4-Dihydro-4-[3-(2-methyl-propyloxy)-2-piperidiono-propyl]-3-(4-propyloxy-phenyl)-2H-1,4-benzoxazin
3,4-Dihydro-4-[3-(2-methyl-propyloxy)-2-pyrrolidino-propyl]-3-(3,4-dimethoxy-phenyl)-2H-1,4-benzothiazin
3,4-Dihydro-4-[3-(2-methyl-2-propenyl-1-oxy)-2-pyrrolidino-propyl]-3-(3,4-dimethoxy-phenyl)-2H-1,4-benzothiazin
3,4-Dihydro-4-[3-(2-methyl-propyloxy)-2-diethylamino-propyl)-3-(4-fluor-phenyl)-2H-1,4-benzothiazin
3,4-Dihydro-4-[3-(2-methyl-2-propenyl-1-oxy)-2-pyrrolidino-propyl]-3-(3,4,5-trimethoxy-phenyl)-2H-1,4-benzothiazin
3,4-Dihydro-4-[3-(2-methyl-propyloxy)-2-pyrrolidino-propyl]-3-(3,4,5-trimethoxy-phenyl)-2H-1,4-benzothiazin
3,4-Dihydro-4-[3-(2-methyl-propyloxy)-2-diethylamino-propyl]-3-(2-methoxy-phenyl)-2H-1,4-benzothiazin
3,4-Dihydro-4-[3-(2-methyl-2-propenyl-1-oxy)-2-pyrrolidino-propyl]-3-(2-methoxy-phenyl)-2H-1,4-benzothiazin
3,4-Dihydro-4-(3-phenoxy-2-morpholino-propyl)-3-(2-methoxy-phenyl)-2H-1,4-benzoxazin
3,4-Dihydro-4-[3-(2-methyl-propyloxy)-2-pyrrolidino-propyl]-3-(3-methoxy-phenyl)-2H-1,4-benzothiazin
3,4-Dihydro-4-[3-(2-methyl-2-propenyl-1-oxy)-2-pyrrolidino-propyl]-3-(3-methoxy-phenyl)-2H-1,4-benzothiazin
3,4-Dihydro-4-[3-(2-methyl-2-propenyl-1-oxy)-2-diethylamino-propyl]-3-(3-methoxy-phenyl)-2H-1,4-benzothiazin
3,4-Dihydro-4-[3-(2,2-dimethyl-propyloxy)-2-diethylamino-propyl]-3-(3-methoxy-phenyl)-2H-1,4-benzothiazin
3,4-Dihydro-4-[3-(2-methyl-2-propenyl-1-oxy)-2-diethylamino-propyl]-3-(4-methoxy-phenyl)-2H-1,4-benzothiazin
3,4-Dihydro-4-[3-(3-methyl-2-butenyl-1-oxy)-2-pyrrolidino-propyl]-3-(4-methoxy-phenyl)-2H-1,4-benzothiazin
3,4-Dihydro-4-[3-(3-methyl-3-butenyl-1-oxy)-2-pyrrolidino-propyl]-3-(4-methoxy-phenyl)-2H-1,4-benzothiazin
3,4-Dihydro-4-[3-(2-methyl-propyloxy)-2-pyrrolidino-propyl]-3-(4-hydroxy-phenyl)-2H-1,4-benzothiazin
3,4-Dihydro-4-[3-(2-methyl-propyloxy)-2-pyrrolidino-propyl]-3-(4-allyloxy-phenyl)-2H-1,4-benzothiazin
3,4-Dihydro-4-[3-(2-methyl-2-propenyl-1-oxy)-2-diethylamino-propyl]-3-(4-allyloxy-phenyl)-2H-1,4-benzothiazin
3,4-Dihydro-4-[3-(2-methyl-propyloxy)-2-pyrrolidino-propyl]-3-(4-propyloxy-phenyl)-2H-1,4-benzothiazin
3,4-Dihydro-4-[3-(2-methyl-2-propenyl-1-oxy)-2-diethylamino-propyl]-3-(4-propyloxy-phenyl)-2H-1,4-benzothiazin
1-[3-(2-Methyl-2-propenyl-1-oxy)-2-pyrrolidino-propyl]-2-(4-methoxy-phenyl)-1,2,3,4-tetrahydro-chinolin
1-[3-(2-Methyl-2-propenyl-1-oxy)-2-diethylamino-propyl]-2-phenyl)-1,2,3,4-tetrahydro-chinolin
1-[3-(2,2-Dimethyl-propyloxy)-2-pyrrolidino-propyl]-2-(4-methoxy-phenyl)-1,2,3,4-tetrahydro-chinolin
1-(3-Phenoxy-2-pyrrolidino-propyl)-2-(4-methoxy-phenyl)-1,2,3,4-tetrahydro-chinolin
1-[3-(2-Methyl-propoxy)-2-(3-methoxy-pyrrolidino)-propyl]-2-(4-methoxy-phenyl)-1,2,3,4-tetrahydro-chinolin
1-[3-(2-Methyl-2-propenyl-2-oxy)-2-(3-methoxy-pyrrolidino)-propyl]-2-(4-methoxy-phenyl)-1,2,3,4-tetrahydro-chinolin
1-[3-(3-Methyl-3-butenyl-1-oxy)-2-pyrrolidino-propyl]-2-(4-methoxy-phenyl)-1,2,3,4-tetrahydro-chinolin
5-[3-(2-Methyl-propyloxy)-2-pyrrolidino-propyl]-4-phenyl-2,3,4,5-tetrahydro-1,5-benzoxazepin
5-[3-(2-Methyl-propyloxy)-2-diethylamino-propyl]-4-phenyl-2,3,4,5-tetrahydro-1,5-benzoxazepin
5-[3-(2-Methyl-2-propenyl-1-oxy)-2-pyrrolidino-propyl]-4-phenyl-2,3,4,5-tetrahydro-1,5-benzoxazepin
5-[3-(2-Methyl-propyloxy)-2-pyrrolidino-propyl]-4-(4-methoxy-phenyl)-2,3,4,5-tetrahydro-1,5-benzoxazepin
5-[3-(2-Methyl-propyloxy)-2-diethylamino-propyl]-4-(4-methoxy-phenyl]-2,3,4,5-tetrahydro-1,5-benzoxazepin
5-[3-(2-Methyl-propyloxy)-2-pyrrolidino-propyl]-4-(4-methoxy-phenyl)-8-methoxy-2,3,4,5-tetrahydro-1,5-benzoxazepin
5-[3-(2-Methyl-propyloxy)-2-pyrrolidino-propyl]-4-(4-fluor-phenyl)-2,3,4,5-tetrahydro-1,5-benzoxazepin
5-[3-(2-Methyl-2-propenyl-1-oxy)-2-diethylamino-propyl]-4-(4-fluor-phenyl)-2,3,4,5-tetrahydro-1,5-benzoxazepin
5-[3-(2,2-Dimethyl-propyloxy)-2-diethylamino-phenyl]-4-(4-fluor-phenyl)-2,3,4,5-tetrahydro-1,5-benzoxazepin
5-[3-(2-Methyl-3-butenyl-1-oxy)-2-pyrrolidino-propyl]-4-(4-fluor-phenyl)-2,3,4,5-tetrahydro-1,5-benzoxazepin
5-[3-(2-Methyl-propyloxy)-2-(3-methoxy-pyrrolidino)-4-phenyl-2,3,4,5-tetrahydro-1,5-benzoxazepin
5-[3-(2-Methyl-propyloxy)-2-(3-methoxy-pyrrolidino)-4-(4-methoxy-phenyl)-2,3,4,5-tetrahydro-1,5-benzoxazepin
5-[3-(2-Methyl-propyloxy)-2-(3-methoxy-pyrrolidino)-4-(4-fluor-phenyl)-2,3,4,5-tetrahydro-1,5-benzoxazepin
5-(3-Phenoxy-2-pyrrolidino-propyl)-4-phenyl-2,3,4,5-tetrahydro-1,5-benzoxazepin
5-[3-(4-Methoxy-phenoxy)-2-(3-methoxy-pyrrolidino)-propyl]-4-(methoxy-phenyl)-2,3,4,5-tetrahydro-1,5-benzoxazepin
5-[3-(4-Methoxy-phenoxy)-2-diethylamino-propyl]-4-(4-methoxy-phenyl)-2,3,4,5-tetrahydro-1,5-benzoxazepin

5-[3-(2-Methyl-propoxy)-2-pyrrolidino-propyl]-4-phenyl-2,3,4,5-tetrahydro-1,5-benzothiazepin
5-[3-(2-Methyl-propoxy)-2-diethylamino-propyl]-4-phenyl-2,3,4,5-tetrahydro-1,5-benzothiazepin
5-[3-(2-Methyl-2-propenyl-1-oxy)-2-pyrrolidino-propyl]-4-phenyl-2,3,4,5-tetrahydro-1,5-benzothiazepin
5-[3-(2,2-Dimethyl-propyloxy)-2-pyrrolidino-propyl]-4-phenyl-2,3,4,5-tetrahydro-1,5-benzothiazepin
5-[3-(2,2-Dimethyl-propyloxy)-2-(3-methoxy-pyrrolidino-propyl]-4-phenyl-2,3,4,5-tetrahydro-1,5-benzothiazepin
5-[3-(2-Methyl-3-butenyl-2-oxy)-2-diethylamino-propyl]-4-phenyl-2,3,4,5-tetrahydro-1,5-benzothiazepin
5-[3-(2-Methyl-propoxy)-2-pyrrolidino-propyl]-4-(4-methoxy-phenyl)-2,3,4,5-tetrahydro-1,5-benzothiazepin
5-[3-(2-Methyl-2-propenyl-1-oxy)-2-diethylamino-propyl]-4-(4-methoxy-phenyl)-2,3,4,5-tetrahydro-1,5-benzothiazepin
5-[3-(2-Methyl-propoxy)-2-pyrrolidino-propyl]-4-(4-fluor-phenyl)-2,3,4,5-tetrahydro-1,5-benzothiazepin
5-(3-Phenoxy-2-pyrrolidino-propyl)-4-phenyl-2,3,4,5-tetrahydro-1,5-benzothiazepin
5-[3-(4-Methoxy-phenoxy)-2-(3-methoxy-pyrrolidino)-propyl]-4-(4-methoxy-phenyl)-2,3,4,5-tetrahydro-1,5-benzothiazepin
5-[3-(4-Methoxy-phenoxy)-2-dimethylamino-propyl]-4-(4-methoxy-phenyl)-2,3,4,5-tetrahydro-1,5-benzothiazepin

Beispiel 1:

1-[3-(2-Methyl-propyloxy)-2-pyrrolidino-propyl]-2-phenyl-indolin

4 g 2-Phenyl-indolin werden in 25 ml Dimethylformamid gelöst und die Lösung mit 1.2 g Natriumhydrid (50proz. Suspension in Öl) versetzt. Man erwärmt unter Rühren auf 80 °C solange, bis die Wasserstoffentwicklung beendet ist. Danach tropft eine Lösung von 4.5 g 1-[2-Chlor-3-(2-methyl-propoxy)-propyl]pyrrolidin in 25 ml Dimethylformamid im Verlauf von 15 min zu und rührt noch 3 Std. bei 80 °C weiter. Man kühlt, versetzt die Reaktionsmischung mit Wasser und extrahiert mit Methylenchlorid. Die Methylenchloridphase wird mit Wasser gewaschen, über Natriumsulfat getrocknet und eingedampft. Der Rückstand wird zur Reinigung an einer Kieselgelsäule chromatographiert. (Elutionsmittel: Toluol/10 % Essigester). Nach Eindampfen der entsprechenden Säulenfraktionen erhält man 3.5 g der Titelverbindung als öliges Produkt.

Analog wurden dargestellt:

Beispiel 2:

1-[3-(2-Methyl-propyloxy)-2-pyrrolidino-propyl]2-(4-methoxy-phenyl)-indolin, Öl m/e 408

Beispiel 3:

3,4-Dihydro-4-[3-(2-methyl-propyloxy)-2-pyrrolidino-propyl]-3-phenyl-2H-1,4-benzoxazin-Oxalat;
Schmp. 159-160 °C

Beispiel 4:

3,4-Dihydro-4-[3-(2-methyl-propyloxy)-2-pyrrolidino-propyl]-3-(4-methoxy-phenyl)-2H-1,4-benzoxazin;
Öl m/e 424

Beispiel 5:

1-[3-(2-Methyl-propyloxy)-2-pyrrolidino-propyl]2-phenyl-1,2,3,4-tetrahydro-chinolin-oxalat
Schmp. 141-142 °C

Beispiel 6:

3,4-Dihydro-4-[3-(2-methyl-2-propenyl-1-oxy)-2-pyrrolidino-propyl]-3-phenyl-2H-1,4-benzoxazin-Oxalat;
Schmp. 120-121 °C

Beispiel 7:

3,4-Dihydro-4-[3-(3-methyl-2-butenyl-1-oxy)-2-pyrrolidino-propyl]-3-phenyl-2H-1,4-benzoxazin; Öl m/e 406

Beispiel 8:

3,4-Dihydro-4-[3-(3-methyl- 3-butenyl-1-oxy)-2-pyrrolidino-propyl]-3-phenyl-2H-1,4-benzoxazin; Öl m/e 406

Beispiel 9:

3,4-Dihydro-4-[3-((1-methylcyclohexyl)methoxy)-2-pyrrolidino-propyl]-3-phenyl-2H-1,4-benzoxazin, Öl m/e 448

Beispiel 10:

3,4-Dihydro-4-[3-(2-methyl-propyloxy)-2-pyrrolidino-propyl]-3-(4-methoxy-phenyl)-7-methoxy-2H-1,4-benzoxazin Öl m/e 454

Beispiel 11:

3,4-Dihydro-4-[3-(2,2-Dimethyl-propyloxy)-2-pyrrolidino-propyl]-3-phenyl-2H-1,4-benzoxazin, Öl m/e 408

Beispiel 12:

3,4-Dihydro-4-[3-(2-methyl-propyloxy)-2-pyrrolidino-propyl]-3-phenyl-2H-1,4-benzothiazin, Öl m/e 410

Beispiel 13:

3,4-Dihydro-4-[3-(2-methyl-3-butenyl-2-oxy)-2-pyrrolidino-propyl]-3-phenyl-2H-1,4-benzoxazin, Öl m/e 406

Beispiel 14:

1-[3-(2-Methyl-3-butenyl-2-oxy)-2-pyrrolidino-propyl]-2-phenyl-indolin, Öl m/e 390

Beispiel 15:

1-[3-(3-Methyl-2-butenyl-1-oxy)-2-pyrrolidino-propyl]-2-phenyl-indolin, Öl m/e 390

Beispiel 16:

1-[3-(3-Methyl-3-butenyl-1-oxy)-2-pyrrolidino-propyl]-2-phenyl-indolin, Öl m/e 390

Beispiel 17:

3,4-Dihydro-4-[3-(2-methyl-propyloxy)-2-diethylamino-propyl]-3-phenyl-2H-1,4-benzoxazin, Öl m/e 396

Beispiel 18:

1-[3-(2-Methyl-propoxy)-2-pyrrolidino-propyl]-4-methyl-2-phenyl-1,2,3,4-tetrahydro-1,4-benzodiazin, Öl m/e 407

Beispiel 19:

1-[3-(2-Methyl-propoxy)-2-pyrrolidino-propyl]-2-(4-methoxy-phenyl)-1,2,3,4-tetrahydro-chinolin, Öl m/e 422

Beispiel 20:

3,4-Dihydro-4-(3-alloxy-2-pyrrolidino-propyl)-3-phenyl-2H-1,4-benzoxazin, Öl m/e 378

Zur Charakterisierung der hergestellen Verbindungen wird in vielen Fällen der massenspektrometrisch bestimmte Molpeak m/e angegeben.

Darstellung der Ausgangsmaterialien

Die Darstellung von 1-[2-Chlor-3-(2-methyl-propoxy)-propyl]pyrrolidin wird als repräşentatives Beispiel beschrieben. Die anderen benötigten Ausgangsmaterialien der allgemeine Formel II wurden auf analogem Wege dargestellt.

1. Stufe:

1-(2-methyl-propoxy)-2,3-epoxypropan

Eine Mischung aus 24.2 g Isobutanol, 85 ml konz. Natronlauge, 100 ml Epichlorhydrin und 2 g Tetrabutoxyamminiumbromid wird 5 Std. bei 40° C intensiv gerührt. Dann wird abgekühlt, die Reaktionsmischung auf Eiswasser gegossen und mit Essigester extrahiert. Die Essigesterphase wird abgetrennt, über Natriumsulfat getrocknet und eingedampft. Der Rückstand wird i. Vak. destilliert.

Sdp. 75-80° C/50 mm.

Man erhält 25 g 1-(2-methyl-propoxy)-2,3-epoxypropan.

Analog:

1-Allyloxy-2,3-epoxy-propan

Sdp. 78° C/56 mm

1-(2-Methyl-2-propenyl-1-oxy)-2,3-epoxy-propan

Sdp.: 84-87° C/40 mm

1-(3-Methyl-2-butenyl-1-oxy)-2,3-epoxy-propan

Sdp: 85-87° C/13 mm

1-(3-Methyl-3-butenyl-1-oxy)-2,3-epoxy-propan

Sdp.: 77-80° C/13 mm

1-(2-Methyl-3-butenyl-2-oxy)-2,3-epoxy-propan

Sdp.: 66-70° C/13 mm

1-[(1-Methylcyclohexyl)methoxy]-2,3-epoxy-propan

Sdp.: 120-125° C/13 mm

1-(2,2-Dimethyl-propoxy)-2,3-epoxy-propan

Sdp.: 85-86° C/50 mm

2. Stufe:

3-(2-Methyl-propoxy)-1-pyrrolidino-propanol-2

20 g 1-(2-Methyl-propoxy)-2,3-epoxy-propan werden in 40 ml Ethanol gelöst. Diese Lösung wird auf 55° C erwärmt. Anschließend tropft man im Verlauf einer Stunde unter Rühren eine Lösung von 13 g Pyrrolidin in 25 ml Ethanol zu. Hierbei soll die Temperatur nicht über 70° C steigen. Nach beendeter Zugabe rührt man noch eine Stunde bei 70° C weiter, dampft ein und destilliert den Rückstand i. Vak..

Sdp. 134° C/14 mm.

Man erhält 35 g 3-(2-Methyl-propoxy)-1-pyrrolidino-propanol-2.

Analog:

3-Allyloxy-1-pyrrolidino-propanol-2

Sdp.: 75° C/$10^{-2}$ mm

3-(2-Methyl-propenyloxy)-1-pyrrolidino-propanol-2

Sdp.: 94° C/$10^{-2}$ mm

3-(2-Methyl-2-butenyl-1-oxy)-1-pyrrolidino-propanol-2

Sdp.: 94° C/$2.10^{-2}$ mm

3-(3-Methyl-3-butenyl-1-oxy)-1-pyrrolidino-propanol-2
Sdp.:108° C/4.10$^{-2}$ mm
3-(2-Methyl-3-butenyl-2-oxy)-1-pyrrolidino-propanol-2
Sdp.: 80° C/2.10$^{-2}$ mm
3-[(1-Methylcyclohexyl)-1-pyrrolidino-propanol-2
Sdp.:107° C/10$^{-2}$ mm
3-(2,2-Dimethyl-propoxy)-1-pyrrolidino-propanol-2
Sdp.: 76° C/10$^{-2}$ mm
3-(2-Methyl-propoxy)-1-diethylamino-propanol-2
Sdp.: 80° C/1.5 mm

3. Stufe:

1-[2-Chlor-3-(2-methyl-propoxy)-propyl]pyrrolidin

10 g 3-(2-Methyl-propoxy)-1-pyrrolidino-propanol-2 werden in 100 ml Dichloroethan gelöst. Hierzu tropft man unter Rühren eine Lösung von 7.1 g Thionylchlorid in 20 ml Dichloroethan und erwärmt anschließend 2 Stdn. auf 70° C. Die Reaktionsmischung wird abgekühlt, auf Eiswasser ge gossen und anschließend durch Zugabe von konz. Natronlauge alkalisch gestellt. Die organische Phase wird abgetrennt und die wäßrige Phase nochmals mit Methylenchlorid extrahiert. Die organischen Phasen werden vereinigt, über Natriumsulfat getrocknet und eingedampft. Der Rückstand wird durch Chromatographie an einer Kieselgelsäule gereinigt Elutionsmittel: Methylenchlorid/5 % Methanol). Man erhält 9 g der Titelverbindung als öliges Produkt.

Analog:
1-(3-(Allyloxy-2-chlor-propyl)pyrrolidin
1-[2-Chlor-(2-methyl-propenyl-1-oxy)propyl]pyrrolidin
1-[2-Chlor-(3-methyl-2-butenyl-1-oxy)propyl]pyrrolidin
1-[2-Chlor-(3-methyl-3-butenyl-1-oxy)propyl]pyrrolidin
1-[2-Chlor-(2-methyl-3-butenyl-1-oxy)propyl]pyrrolidin
1-[2-Chlor-((1-methylcyclohexyl)methoxy)-1-propyl]pyrrolidin
1-[2-Chlor-(2,2-Dimethyl-propoxy)propyl]pyrrolidin
N-[2-Chlor-(2-methyl-propoxy)propyl]-N,N-diethylamin

## Ansprüche

1. Verbindungen der allgemeinen Formel I

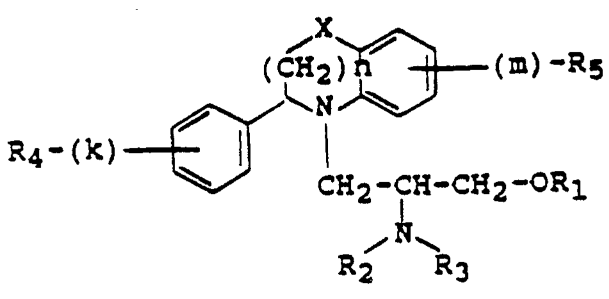

(I)

worin

$R_1$ einen geradkettigen oder verzweigten $C_1$-$C_{12}$-Alkylrest, der durch einen Phenyl-, Naphthyl- oder einen gegebenenfalls durch $C_1$-$C_4$-Alkyl substituierten $C_3$-$C_7$-Cycloalkylrest substituiert sein kann, einen geradkettigen oder verzweigten $C_2$-$C_{12}$-Alkenylrest, der durch einen $C_3$-$C_7$-Cycloalkylrest oder einen Phenyl- oder Naphthyl substituiert sein kann, einen $C_3$-$C_7$-Cycloalkylrest oder einen unsubstituierten oder ein oder mehrfach substituierten mono- oder bicyclischen aromatischen Rest bedeutet,
wobei die Substituenten $C_1$-$C_4$-Alkyl, $C_1$-$C_4$-Alkoxy, Halogen, Carboxyl oder $C_1$-$C_4$-Alkoxycarbonyl sein können,

$R_2$ und $R_3$ gleich oder verschieden sein können und einen geradkettigen, verzweigten, gesättigten oder ungesättigten $C_1$-$C_6$ Alkylrest bedeuten oder zusammen mit dem Stickstoff einen gesättigten oder ungesättigten Ring bilden, der noch weitere Heteroatome enthalten kann und gegebenenfalls durch eine niedrige $C_1$-$C_6$ Alkylgruppe, eine oder mehrere $C_1$-$C_6$ Alkoxygruppen, durch eine oder mehrere Hydroxylgruppen oder ein Sauerstoffatom substituiert sein kann,

X eine Methylengruppe, ein Sauerstoffatom, ein Schwegelatom, die Sulfoxidgruppe, die Sulfonylgruppe oder die Gruppe $NR_6$ bedeutet, wobei $R_6$ Wasserstoff, $C_1$-$C_4$ Alkyl oder Aralkyl bedeuten kann,

n die Bedeutung 0, 1 oder 2 besitzt,

k die Bedeutung 0, 1, 2 oder 3 besitzt,

$R_4$ Halogen, $C_1$-$C_6$ Alkyl, $C_1$-$C_6$ Alkoxy, Aralkoxy, Hydroxy oder wenn k gleich 2 ist an benachbarten Kohlenstoffatomen $C_1$-$C_2$ Alkylendioxy bedeutet

m die Bedeutung 0, 1, 2 oder 3 besitzt und

$R_5$ Halogen, $C_1$-$C_4$ Alkyl, $C_1$-$C_6$ Alkoxy, Aralkoxy, Hydroxy oder wenn m = 2 ist an benachbarten Kohlenstoffatomen $C_1$-$C_2$ Alkylendioxy bedeutet.

sowie deren pharmakolgisch verträglichen Salze und optische Isomere.

2. Bevorzugt sind Verbindungen der Formel I, in der

$R_1$ Isobutyl, Isoamyl, Isohexyl, Neopentyl, 1-Methylcyclohexylmethyl, Allyl, Methallyl, Isopentyl, Hexenyl, Octenyl, Dodecenyl oder Cinnamyl,

$R_2$ und $R_3$ jeweils Ethyl oder zusammen mit dem Stickstoffatom, an das sie gebunden sind, einen Pyrrolidin-Ring bilden,

X eine Methylengruppe, ein Sauerstoff- oder Schwefelatom, oder die Gruppe NH,

K die Zahlen 0 oder 1,

m die Zahlen 0 oder 1,

n die Zahlen 0 oder 1,

$R_4$ Methoxy und

$R_5$ Methoxy

bedeuten.

3. Verfahren zur Herstellung von Verbindungen der Formel

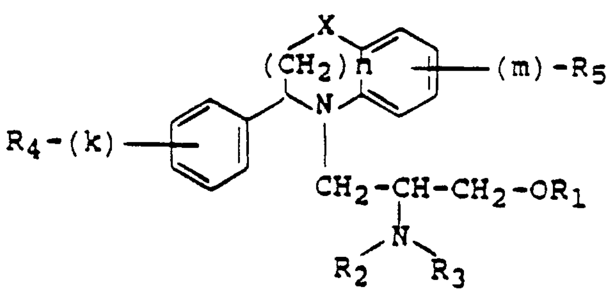

(I)

worin

$R_1$ einen geradkettigen oder verzweigten $C_1$-$C_{12}$-Alkylrest, der durch einen Phenyl-, Naphthyl- oder einen gegebenenfalls durch $C_1$-$C_4$-Alkyl substituierten $C_3$-$C_7$-Cycloalkylrest substituiert sein kann, einen geradkettigen oder verzweigten $C_2$-$C_{12}$-Alkenylrest, der durch einen $C_3$-$C_7$-Cycloalkylrest oder einen Phenyl- oder Naphthyl substituiert sein kann, einen $C_3$-$C_7$-Cycloalkylrest oder einen unsubstituierten oder ein- oder mehrfach substituierten mono- oder bicyclischen aromatischen Rest bedeutet,

wobei die Substituenten $C_1$-$C_4$-Alkyl, $C_1$-$C_4$-Alkoxy, Halogen, Carboxyl oder $C_1$-$C_4$-Alkoxycarbonyl sein können,

$R_2$ und $R_3$ gleich oder verschieden sein können und einen geradkettigen, verzweigten, gesättigten oder ungesättigten $C_1$-$C_6$ Alkylrest bedeuten oder zusammen mit dem Stickstoff einen gesättigten oder ungesättigten Ring bilden, der noch weitere Heteroatome enthalten kann und gegebenenfalls durch eine niedrige $C_1$-$C_6$ Alkylgruppe, eine oder mehrere $C_1$-$C_6$ Alkoxygruppen, durch eine oder mehrere Hydroxylgruppen oder ein Sauerstoffatom substituiert sein kann,

X eine Methylengruppe, ein Sauerstoffatom, ein Schwefelatom, die Sulfoxidgruppe, die Sulfonylgruppe oder die Gruppe $NR_6$ bedeutet, wobei $R_6$ Wasserstoff, $C_1$-$C_4$ Alkyl oder Aralkyl bedeuten kann,

n die Bedeutung 0, 1 oder 2 besitzt,

k die Bedeutung 0, 1, 2 oder 3 besitzt,

$R_4$ Halogen, $C_1$-$C_6$ Alkyl, $C_1$-$C_6$ Alkoxy, Aralkoxy, Hydroxy oder wenn k gleich 2 ist an benachbarten Kohlenstoffatomen $C_1$-$C_2$ Alkylendioxy bedeutet

m die Bedeutung 0, 1, 2 oder 3 besitzt und
$R_5$ Halogen, $C_1$-$C_4$ Alkyl, $C_1$-$C_6$ Alkoxy, Aralkoxy, Hydroxy oder wenn m = 2 ist an benachbarten Kohlenstoffatomen $C_1$-$C_2$ Alkylendioxy bedeutet.
sowie deren pharmakologisch unbedenklichen Salze und optische Isomere,
dadurch gekennzeichnet, daß man in üblicher Weise
a) eine Verbindung der allgemeinen Formel II,

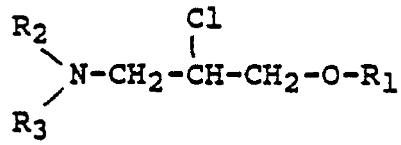

(II)

in der $R_1$, $R_2$, und $R_3$ die oben genannten Bedeutungen besitzen, mit einer Verbindung der allgemeinen Formel III,

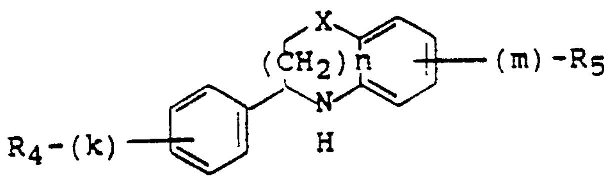

(III)

in der $R_4$, $R_5$, k, m und X die oben genannten Bedeutungen besitzen, umsetzt
mit der Ausnahme, daß man, wenn die beiden Reste $R_2$ und $R_3$ zusammen einen durch eine oder zwei Hydroxylgruppen substituierten Ring bilden oder einer der Reste $R_4$ oder $R_5$ eine Hydroxylgruppe darstellt, diese vor der Umsetzung schützt und nach beendeter Reaktion wieder abspaltet. Als Schutzgruppen kommen z.B. in Frage Benzyloxygruppen oder Methoxy-methylenoxy-gruppen.
oder
b) eine Verbindung der allgemeinen Formel IV,

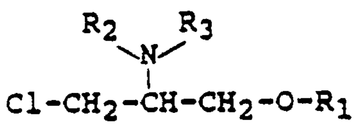

(IV)

in der $R_1$, $R_2$, und $R_3$ die oben genannten Bedeutungen besitzen, mit einer Verbindung der allgemeinen Formel III umsetzt,
wobei die gleichen Ausnahmen, wie unter a) beschrieben, gelten.
oder
c) eine Verbindung der allgemeinen Formel V,

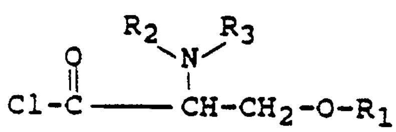

(V)

in der $R_1$, $R_2$, und $R_3$ die oben genannten Bedeutungen besitzen mit einer Verbindung der allgemeinen Formel III umsetzt und die erhaltene Verbindung der allgemeinen Formel VI,

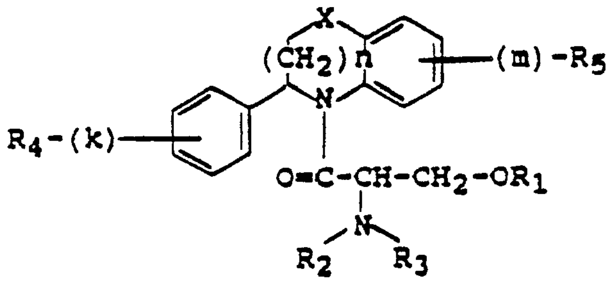

(VI)

in der $R_1$, $R_2$, $R_3$, $R_4$, $R_5$, X, k, m und n die oben genannten Bedeutungen, einer Reduktion mit einem komplexen Hydrid unterwirft.
und anschließend gewünschtenfalls die erhaltenen Verbindungen in ihre pharmakologisch unbedenklichen Salze oder optische Isomere überführt.

4. Arzneimittel, enthaltend eine Verbindung gemäß Anspruch 1 oder 2 neben üblichen Träger- und Hilfsstoffen.

5. Verwendung von Verbindungen gemäß Anspruch 1 oder 2 zur Behandlung von Herz- und Kreislauferkrankungen.

Europäisches Patentamt

# EUROPÄISCHER RECHERCHENBERICHT

Nummer der Anmeldung: EP 89104064.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/A3 - 0 166 591 (MERCK FROSST CANADA INC.)<br>* Zusammenfassung; Seiten 1,2, 70-72 *<br>-- | 1,4,5 | C 07 D 207/00<br>C 07 D 209/22<br>C 07 D 223/16<br>C 07 D 215/22<br>C 07 D 235/06<br>C 07 D 277/64<br>C 07 D 279/16<br>C 07 D 265/36<br>C 07 D 241/42<br>C 07 D 243/12 |
| A | EP - A1 - 0 229 329 (E.R. SQUIBB & SONS, INC.)<br>* Zusammenfassung; Ansprüche 1, 35,38 *<br>-- | 1,3-5 | |
| A | US - A - 4 225 430 (HERBERT KÖPPE et al.)<br>* Patentanspruch 1; Zusammenfassung *<br>---- | 1,4 | |
| | | | **RECHERCHIERTE SACHGEBIETE (Int. Cl.4)** |
| | | | C 07 D 207/00<br>C 07 D 209/00<br>C 07 D 215/00<br>C 07 D 223/00<br>C 07 D 235/00<br>C 07 D 277/00<br>C 07 D 279/00<br>C 07 D 265/00<br>C 07 D 241/00<br>C 07 D 243/00 |

Der vorliegende Recherchenbericht wurde für alle Patentansprüche erstellt.

| Recherchenort | Abschlußdatum der Recherche | Prüfer |
|---|---|---|
| WIEN | 30-05-1989 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82